# EUROPEAN PATENT APPLICATION

(11) **EP 2 213 735 A1**
(43) Date of publication of application: **04.08.2010**
(21) Application number: 08846313.8
(22) Date of filing: 20.10.2008
(51) Int. Cl.: C12N 15/09, A61K 9/127, A61K 39/395, A61K 47/42, C07K 14/005, C07K 14/31, C07K 19/00

(54) **PEPTIDE IMPARTING ANTIBODY-BINDING ABILITY TO LIPOSOME AND LIPOSOME MODIFIED WITH THE SAME**

(30) Priority: 06.11.2007 JP 2007288728
(71) Applicant: The University of Tokushima, Tokushima-shi Tokushima 770-8501 (JP)
(72) Inventor: SHINOHARA, Yasuo, Tokushima-shi Tokushima 770-8503 (JP); KIWADA, Hiroshi, Tokushima-shi Tokushima 770-8505 (JP); ISHIDA, Tatsuhiro, Tokushima-shi Tokushima 770-8505 (JP); YAMAZAKI, Naoshi, Tokushima-shi Tokushima 770-8505 (JP); KATAOKA, Masatoshi, Takamatsu-shi Kagawa 761-0395 (JP)
(74) Representative: Müller-Boré & Partner Patentanwälte
(86) International application number: PCT/JP2008/068972
(87) International publication number: WO 2009/060710

(57) **Abstract**

The present invention provides a liposome having antibody-binding ability and a preparation method thereof, and an antibody-modified liposome and a preparation method thereof. The antibody-binding liposome of the invention is **characterized by** having a surface modified with a peptide having an amino acid sequence represented by SEQ ID NO: 1, or a peptide functionally equivalent thereto. Moreover, the antibody-modified liposome of the invention is **characterized by** being obtained by binding an antibody to the above antibody-binding liposome via the peptide having the amino acid sequence of SEQ ID NO: 1, or a peptide functionally equivalent thereto.

SEQUENCE LISTING

<110>  THE UNIVERSITY OF TOKUSHIMA
<110>  NATIONAL INSTITUTE OF ADVANCED INDUSTRIAL SCIENCE AND TECHNOLOGY

<120>  Peptide for providing antibody binding ability to liposome, and liposome
modified by the peptide

<130>  P08-112

<150>  JP 2007-288728
<151>  2007-11-6

<160>  6

<170>  PatentIn version 3.4

<210>  1
<211>  138
<212>  PRT
<213>  Artificial Sequence
<220>
<223>  Linker peptide
<400>  1
Met Ala Gln His Asp Glu Ala Ser Ala Asp Asn Lys Phe Asn Lys Glu
1               5                   10                  15
Gln Gln Asn Ala Phe Tyr Glu Ile Leu His Leu Pro Asn Leu Asn Glu
            20                  25                  30
Glu Gln Arg Asn Gly Phe Ile Gln Ser Leu Lys Asp Asp Pro Ser Gln
        35                  40                  45
Ser Ala Asn Leu Leu Ala Glu Ala Lys Lys Leu Asn Asp Ala Gln Ala
    50                  55                  60
Pro Lys Glu Gln Lys Leu Ile Ser Glu Glu Lys Gln Ser Val Ile Thr
65                  70                  75                  80
Asp Val Thr Gly Gln Leu Thr Ala Val Gln Ala Asp Ile Thr Thr Ile
                85                  90                  95
Gly Gly Ala Ile Leu Leu Leu Ile Val Leu Ala Ala Val Val Leu Gly
            100                 105                 110
Ile Arg Trp Ile Lys Ala Gln Phe Phe Met Ala Ser Met Thr Gly Gly
        115                 120                 125
Gln Gln Met Gly His His His His His His
    130                 135

<210>  2
<211>  73
<212>  PRT
<213>  Artificial Sequence
<220>
<223>  Liposome-binding peptide
<400>  2
Met Glu Gln Lys Leu Ile Ser Glu Glu Lys Gln Ser Val Ile Thr Asp
1               5                   10                  15
Val Thr Gly Gln Leu Thr Ala Val Gln Ala Asp Ile Thr Thr Ile Gly
            20                  25                  30
Gly Ala Ile Leu Leu Leu Ile Val Leu Ala Ala Val Val Leu Gly Ile
        35                  40                  45
Arg Trp Ile Lys Ala Gln Phe Phe Met Ala Ser Met Thr Gly Gly Gln

50                  55                  60
Gln Met Gly His His His His His His
65                  70

<210>  3
<211>  66
<212>  PRT
<213>  Artificial Sequence
<220>
<223>  Antibody-binding peptide
<400>  3
Met Ala Gln His Asp Glu Ala Ser Ala Asp Asn Lys Phe Asn Lys Glu
1               5                   10                  15
Gln Gln Asn Ala Phe Tyr Glu Ile Leu His Leu Pro Asn Leu Asn Glu
            20                  25                  30
Glu Gln Arg Asn Gly Phe Ile Gln Ser Leu Lys Asp Asp Pro Ser Gln
        35                  40                  45
Ser Ala Asn Leu Leu Ala Glu Ala Lys Lys Leu Asn Asp Ala Gln Ala
    50                  55                  60
Pro Lys
65

<210>  4
<211>  8
<212>  PRT
<213>  Artificial Sequence
<220>
<223>  His-taq peptide
<400>  4
Ala Ser His His His His His His
                5

<210>  5
<211>  74
<212>  PRT
<213>  Artificial Sequence
<220>
<223>  SPA peptide
<400>  5
Met Ala Gln His Asp Glu Ala Ser Ala Asp Asn Lys Phe Asn Lys Glu
1               5                   10                  15
Gln Gln Asn Ala Phe Tyr Glu Ile Leu His Leu Pro Asn Leu Asn Glu
            20                  25                  30
Glu Gln Arg Asn Gly Phe Ile Gln Ser Leu Lys Asp Asp Pro Ser Gln
        35                  40                  45
Ser Ala Asn Leu Leu Ala Glu Ala Lys Lys Leu Asn Asp Ala Gln Ala
    50                  55                  60
Pro Lys Ala Ser His His His His His His
65                  70

<210>  6
<211>  138
<212>  PRT
<213>  Artificial Sequence
<220>
<223>  SPA-DR peptide
<400>  6

Met Ala Gln His Asp Glu Ala Ser Ala Asp Asn Lys Phe Asn Lys Glu
1               5                   10                  15
Gln Gln Asn Ala Phe Tyr Glu Ile Leu His Leu Pro Asn Leu Asn Glu
            20                  25                  30
Glu Gln Arg Asn Gly Phe Ile Gln Ser Leu Lys Asp Asp Pro Ser Gln
        35                  40                  45
Ser Ala Asn Leu Leu Ala Glu Ala Lys Lys Leu Asn Asp Ala Gln Ala
    50                  55                  60
Pro Lys Glu Gln Lys Leu Ile Ser Glu Glu Lys Gln Ser Val Ile Thr
65                  70                  75                  80
Asp Val Thr Gly Gln Leu Thr Ala Val Gln Ala Asp Ile Thr Thr Ile
                85                  90                  95
Gly Gly Ala Ile Leu Leu Leu Ile Val Leu Ala Ala Val Val Leu Gly
            100                 105                 110
Ile Arg Trp Ile Lys Ala Gln Phe Phe Met Ala Ser Met Thr Gly Gly
        115                 120                 125
Gln Gln Met Gly His His His His His His
    130                 135

## Description

### TECHNICAL FIELD

The present invention relates to a liposome having antibody-binding ability. More specifically, the present invention relates a liposome modified with a peptide having antibody-binding ability (hereinafter referred to as "antibody-binding liposome"), and a method of preparing the same. The present invention also relates to a liposome binding to an antibody (hereinafter referred to as "antibody-modified liposome") obtained by using the antibody-binding liposome, and a method of preparing the same.

Moreover, the present invention relates to a peptide having an ability to bind to both an antibody and liposome (hereinafter also referred to as "linker peptide") to be used for the modification of liposomes, and a liposome-binding peptide (a peptide having an ability to bind to a liposome) to be used for the preparation of the linker peptide.

### BACKGROUND ART

A liposome is a vesicle composed of an artificial lipid bilayer membrane, and is used as a cell model in basic research. In addition, much attention has been given to the use of liposomes as drug delivery carriers (drug delivery tools) for delivering substances encapsulated in liposomes to specific tissues or cells in an organism. If it is possible to administer liposomes containing drugs, such as anticancer drugs, to an organism, so that they are selectively delivered to specific cells or tissues (e.g., cancer cells), targeted drugs can be incorporated only into specific cells or tissues. Thus, liposomes are expected to greatly contribute to improving the selective toxicity of drugs.

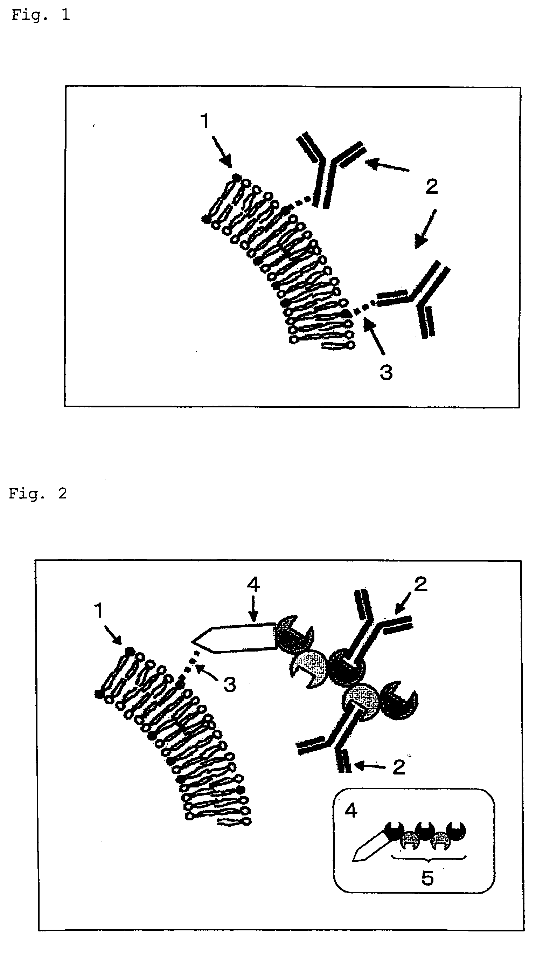

For the purpose of selectively delivering liposomes to specific cells or tissues, the surface of liposomes is often modified with antibodies. In a conventional method of modifying the liposome surface with antibodies, specific antibody-reactive phospholipids are mixed beforehand during the preparation of liposomes, so that antibodies are linked to the resultant liposomes (for example, see Fig. 1). However, this method generally has drawbacks in that the use of specific phospholipids (1) and the preparation of purified antibodies (2) are essential; and that the binding sites of antibody molecules, to which phospholipids bind, cannot be controlled (3).

Further, there has been proposed a method of imparting antibody-binding ability to liposomes by the covalent coupling of Protein A to the liposomes (for example, see Non-Patent Document 1 and Fig. 2). Although this method overcomes the above problem (2) that antibodies must be purified in advance, the problem (1) remains unresolved because it is necessary to preliminarily process phospholipids, which constitute liposomes, so as to have reactivity with Protein A. Additionally, there is another problem in that Protein A, which is a relatively large molecule (molecular weight: about 57 kDa), is difficult to handle. Non-Patent Document 1: Lee D. Leserman, Patrick Machy & Jacques Barbet, "Cell-specific drug transfer from liposomes bearing monoclonal antibodies", Nature, 293 (1981) 226

### DISCLOSURE OF THE INVENTION

### PROBLEMS TO BE SOLVED BY THE INVENTION

The present invention is intended to solve the foregoing problems (1) to (3) in the preparation of "antibody-modified liposomes" by modifying the liposome surface with antibodies. More specifically, an object of the invention is to provide a method of preparing "antibody-modified liposomes" without the use of specific phospholipids (1) and purified antibodies (2), and with the binding sites of antibodies controlled to be predetermined sites (3); and also provide "antibody-binding liposomes" to be suitably used in the preparation of the "antibody-modified liposomes". Another object of the invention is to provide peptides (linker peptide and liposome-binding peptide) to be used in the preparation of the "antibody-binding liposomes".

### MEANS FOR SOLVING THE PROBLEMS

In order to solve the above problems, the present inventors made various improvements based on the amino acid sequence of Pf3 peptide, which is one coat protein of bacteriophage, and successfully formed peptides that have the characteristic of sticking in lipid bilayer membranes, which constitute liposomes, with a specific orientation (migration property and binding property) (see Experimental Example 1). The inventors believed that these peptides (referred to as "liposome-binding peptides" in the present invention) were able to be effectively used as ligands that modify the surface of liposomes.

On the other hand, Protein A, which is produced by *Staphylococcus aureus* and has a high affinity to antibody molecules of many animal species, is composed of five antibody-binding domains comprising about 60 amino acids. Upon selecting one antibody-binding unit (domain B) from these domains and fusing this unit with the liposome-binding peptides, it was confirmed that the resulting fusion peptides spontaneously migrated to liposomes in accordance with the characteristics (migration property and binding property) of the liposome-binding peptides, and bound to the lipid bilayer membranes with the antibody-binding unit (domain B) exposed on the surface of the membranes, thereby imparting antibody-binding ability to the liposomes.

The inventors believed from these findings that the liposomes coupled with the fusion peptides have antibody-binding ability, and can be used as linker peptides to readily produce antibody-modified liposomes. The present invention was thus completed. More specifically, the invention includes the following embodiments.

### (I) Liposome-Binding Peptide

(I-1) A liposome-binding peptide consisting of an amino acid sequence (a) or (b):
(a) an amino acid sequence represented by SEQ ID NO: 2,
   or
(b) an amino acid sequence derived from the amino acid sequence (a) by deletion, substitution or addition of one or several amino acids with the proviso that the amino acid sequence contains at least 11 to 58 and 70 to 73 amino acid regions of the amino acid sequence (a), and encoding a peptide that has a liposome-binding property and a property of migrating to a liposome membrane.

### (II) Antibody-Binding Peptide

(II-1) An antibody-binding peptide consisting of an amino acid sequence (c) or (d):
(c) an amino acid sequence represented by SEQ ID NO: 3,
   or
(d) an amino acid sequence derived from the amino acid sequence (c) by deletion, substitution or addition of one or several amino acids, and encoding a peptide that has antibody-binding ability.

### (III) Linker Peptide

(III-1) A linker peptide consisting of an amino acid sequence (e) or (f):
   (e) an amino acid sequence represented by SEQ ID NO: 1,
      or
   (f) an amino acid sequence derived from the amino acid sequence (e) by deletion, substitution or addition of one or several amino acids, and encoding a peptide that has a liposome-binding property and an antibody-binding property.
(III-2) The linker peptide according to (III-1), in which the liposome-binding peptide of (I-1) binds to the C-terminus of the antibody-binding peptide of (II-1).
(III-3) The linker peptide according to (III-1), in which the liposome-binding peptide of (c) or (d) of (I-1) binds to the C-terminus of the antibody-binding peptide of (e) of (II-1).
(III-4) The linker peptide according to (III-1) obtained by binding the liposome-binding peptide of (c) or (d) of (I-1) to the C-terminus of the antibody-binding peptide of (d) of (II-1).

(IV) Antibody-Binding Liposome and Preparation Method Thereof
(IV-1) An antibody-binding liposome modified with the linker peptide of (III-1) or (III-2).
(IV-2) A method of preparing the antibody-binding liposome according to (IV-1), comprising the step of mixing the linker peptide of (III-1) or (III-2) with a liposome, thereby binding said linker peptide to the liposome.

### (V) Antibody-Modified Liposome and Preparation Method Thereof

(V-1) An antibody-modified liposome in which an antibody binds to the antibody-binding liposome of (IV-1) via the linker peptide of (III-1) or (III-2).
(V-2) The antibody-modified liposome according to (V-1), wherein the antibody is IgG.
(V-3) A method of preparing the antibody-modified liposome according to (V-1) or (V-2), comprising the step of mixing the antibody-binding liposome of (IV-1) with an antibody, thereby binding the antibody to said liposome via the linker peptide of (III-1) or (III-2).

### EFFECT OF THE INVENTION

The linker peptides of the present invention can easily impart antibody-binding ability to arbitrarily prepared liposomes. Particularly, without preliminarily incorporating phospholipids etc. that have been made reactive with proteins into liposomes, as in conventional methods, antibody-binding ability can easily be imparted to liposomes only by mixing the linker peptides with the liposomes.

Conventionally, in coupling antibodies to liposomes, antibodies must be purified before being added to liposomes; however, the antibody-binding liposomes of the invention have specific binding properties to antibodies, particularly to IgG, and therefore the liposomes of the invention can be easily modified with antibodies (IgG) only by directly adding antibodies, such as antiserum, to the liposomes.

In conventional methods, antibodies are nonspecifically bound to liposomes by chemical reaction using a crosslinking agent etc., which may cause deactivation of the antibodies. This problem is avoidable in the method of the present invention, which does not use a crosslinking agent.

Moreover, the antibody-binding unit (domain B) of Protein A, which is used as an antibody-binding domain in the linker peptide of the invention, is known to bind to a hinge region between C_{H}2 and C_{H}3 of the Fc domain of an antibody molecule (Current Opinion in Structural Biology, 1995, 5:471-481). Thus, the antibody-binding liposome of the invention, to which the linker peptide is linked, binds to antibody molecules via the above predetermined site, thereby solving the problem of conventional methods in which the binding sites of antibody molecules, to which phospholipids bind, cannot be controlled.

### BEST MODE FOR CARRYING OUT THE INVENTION

### (1) Antibody-Binding Liposome and Preparation Method Thereof

The "antibody-binding liposome" intended by the present invention is a liposome that has an ability to bind to antibodies.

The "antibody" used herein may be antiserum, and is preferably IgG. The origin of such antibodies is not limited; the antibody-binding liposome of the invention has an ability to bind to antibodies, particularly IgG, derived from any animal species.

The antibody-binding liposome of the invention is characterized by having a surface modified with a peptide consisting of an amino acid sequence represented by SEQ ID NO: 1 (hereinafter referred to as "linker peptide").

The linker peptide consisting of the amino acid sequence represented by SEQ ID NO: 1 is a fusion peptide in which a 2 to 75 amino acid region of the liposome-binding peptide consisting of an amino acid sequence represented by SEQ ID NO: 2 is coupled to the C-terminal side of the antibody-binding peptide consisting of an amino acid sequence represented by SEQ ID NO: 3.

Here, the liposome-binding peptide of SEQ ID NO: 2 is characterized by having a property of spontaneously migrating to a lipid bilayer membrane, which forms a liposome (migration property), and a property of binding to the lipid bilayer membrane in a state where the peptide is stuck in the membrane with a specific orientation, as shown in Experimental Example 1, described later.

The liposome-binding peptide is not limited to the peptide having the specific amino acid sequence represented by SEQ ID NO: 2; a peptide functionally equivalent thereto may also be used. Such a functionally equivalent peptide may be a peptide having an amino acid sequence derived from the amino acid sequence of SEQ ID NO: 2 by deletion, substitution or addition of one or several amino acids, and having the above-described properties of migrating and binding to a liposome (lipid bilayer membrane).

A more preferable functionally equivalent peptide is a peptide having at least 11 to 58 and 70 to 73 amino acid regions of the amino acid sequence of SEQ ID NO: 2. The reasons for this are that even when the first amino acid residue (Met) of the amino acid sequence of SEQ ID NO: 2 is removed, and the amino acid sequence of SEQ ID NO: 3 is coupled thereto, the properties of migrating and binding to a lipid bilayer membrane are not impaired (see Experimental Example 2); and that a 1 to 10 amino acid region and a 59 to 69 amino acid region of the amino acid sequence of SEQ ID NO: 2 respectively correspond to a Myc-tag region and a T7-tag region, which are unrelated to the above-mentioned properties.

Moreover, as shown in Experimental Example 1, described later, the liposome-binding peptide (SEQ ID NO: 2) binds to a lipid bilayer membrane with the C-side region stuck in the lipid bilayer membrane, and the N-side region exposed on the surface of the lipid bilayer membrane. This suggests that the His-tag region in the C-terminus of the amino acid sequence of SEQ ID NO: 2 is involved in the properties of migrating and binding to a liposome (lipid bilayer membrane).

More specifically, examples of the functional equivalent of the peptide of SEQ ID NO: 2 intended by the present invention may include a peptide having any amino acid residues or amino acid sequences in the N-terminal side, a 1-10 region or a 59-69 region of the peptide of SEQ ID NO: 2, and a peptide in which one or several amino acids in these regions are deleted or substituted by other amino acids, insofar as they have at least 11 to 58 and 70 to 73 amino acid regions of the sequence of SEQ ID NO: 2, and the above-described properties of migrating and binding to liposomes.

The liposome-binding property of this peptide can be measured and evaluated according to the methods described in Experimental Examples 1 and 2.

Since the liposome-binding peptide has the properties of specifically migrating to a lipid bilayer membrane and binding thereto, the peptide itself is useful as, for example, a sensitive detection tool for lipid bilayer membranes. Moreover, since the liposome-binding peptide can be fused with a peptide having antibody-binding ability to easily produce a linker peptide that has both a liposome-binding property and an antibody-binding property, the liposome-binding peptide can be effectively used as a preparation tool (preparation material) for such linker peptides.

The antibody-binding peptide of SEQ ID NO: 3 is characterized by having an ability to bind to the above-mentioned antibody, preferably IgG.

The antibody-binding peptide of SEQ ID NO: 3 is derived from one antibody-binding unit (domain B) of Protein A. The antibody-binding peptide is not limited to the peptide having the specific amino acid sequence of SEQ ID NO: 3, and a peptide functionally equivalent thereto may also be used. Such a functionally equivalent peptide may be a peptide having an amino acid sequence derived from the amino acid sequence of SEQ ID NO: 3 by deletion, substitution or addition of one or several amino acids, and having the above-described antibody-binding ability.

A more preferable functionally equivalent peptide is a peptide having the amino acid sequence of SEQ ID NO: 3 modified by the addition of one or several amino acid sequences to the C-terminus thereof. The reason for this is that when a 2 to 73 amino acid region of the amino acid sequence of the liposome-binding peptide represented by SEQ ID NO: 2 is coupled to the C-terminus of the amino acid sequence of SEQ ID NO: 3, the antibody-binding ability is not impaired (see Experimental Example 3). That is, examples of the functional equivalent of the peptide of SEQ ID NO: 3 intended by the present invention may include peptides in which any amino acid residue or amino acid sequence is added to the C-terminus of the amino acid sequence, insofar as they comprise the amino acid sequence of SEQ ID NO: 3 and have antibody-binding ability.

Examples of the functionally equivalent peptide may also include peptides that have an amino acid sequence derived from the amino acid sequence of SEQ ID NO: 3 by deletion or substitution of one or more amino acids. Such a functionally equivalent peptide is preferably a peptide that has an amino acid sequence with an identity of 85% or higher, preferably 90% or higher, and more preferably 95% or higher, to the amino acid sequence of SEQ ID NO: 3; and that has the above-mentioned antibody-binding ability.

The antibody-binding property of this peptide can be measured and evaluated according to the method described in Experimental Example 3.

From the above, the linker peptide is not limited to the peptide consisting of the amino acid sequence of SEQ ID NO: 1. Examples of the linker peptide include peptides functionally equivalent thereto that have a liposome-binding property and an antibody-binding property, and that are each derived from the above-described liposome-binding peptide or antibody-binding peptide.

Examples of such functional equivalents of the linker peptide represented by SEQ ID NO: 1 include a peptide in which the above-described functional equivalent of the liposome-binding peptide of SEQ ID NO: 2 is fused to the C-terminal region of the antibody-binding peptide of SEQ ID NO: 3, and a peptide in which the liposome-binding peptide represented by SEQ ID NO: 2 or a functional equivalent thereof is fused to the C-terminal region of the above-described functional equivalent of the antibody-binding peptide of SEQ ID NO: 3. A preferred example of the functionally equivalent peptides may be a peptide that comprises an amino acid sequence with an identity of at least 95% to the amino acid sequence of SEQ ID NO: 3, as the amino acid sequence of the "antibody-binding peptide", and has at least 11 to 58 and 70 to 73 amino acid regions of the amino acid sequence of SEQ ID NO: 2, as the amino acid sequence of the "liposome-binding peptide"; that consists of an amino acid sequence with an identity of preferably 85% or higher, more preferably 90%, and particularly preferably 95% or higher, to the amino acid sequence of SEQ ID NO: 1; and that has both a liposome-binding property and an antibody-binding property.

These peptides can be produced according to a common use by a scientific or genetic engineering technique on the basis of the amino acid sequences disclosed in the present invention. The modification of amino acid sequences can be carried out using a method already known in the art, e.g., a site-specific mutation induction method (Current Protocols in molecular Biology edit. Ausubel et al. (1987), Publish. John Wily & Sons Section 8.1-8.5), by suitably inducing mutation, e.g., substitution, deletion, insertion or addition, into an amino acid sequence to be modified.

The liposome-binding property of the peptides prepared in this manner can be measured and evaluated according to the methods described in Experimental Example 1 and 2, and the antibody-binding property of the peptides can be measured and evaluated according to the method described in Experimental Example 3.

A liposome is a closed vesicle comprising a phospholipid bilayer membrane and having a structure to form a space isolated from the outside by means of the membrane produced on the basis of the polarity between hydrophobic and hydrophilic groups of the lipid. The aqueous phases inside and outside the closed vesicle across the membrane are referred to as an internal aqueous phase and external aqueous phase, respectively.

The phospholipid, which is a substrate of a liposome membrane, is a main constituent of biomembranes, and is an amphiphile that has a hydrophobic group comprising a long chain alkyl group, and a hydrophilic group comprising a phosphate group, in the molecule. Any natural or synthetic phospholipids can be used as long as they can form liposomes having the above structure. Examples thereof include phosphatidylcholine (also referred to as "lecithin"), phosphatidylethanolamine (abbr.: PE), phosphatidic acid, phosphatidylserine, phosphatidylinositol, phosphatidylglycerol, sphingophospholipids such as sphingomyelin, cardiolipin, and other natural or synthetic phospholipids or derivatives thereof, derivatives binding to saccharides (glycolipids) and hydrogen additives (saturated phospholipids) thereof, and the like.

Specific examples of saturated phospholipids include hydrogen additives such as phosphatidylcholine, phosphatidylethanolamine, phosphatidic acid, phosphatidylserine, phosphatidylinositol, phosphatidylglycerol, and sphingomyelin. Particularly preferred examples of saturated phospholipids are saturated phospholipids derived from egg yolk or soybean, and having a hydrogen addition rate of 50% or more.

These phospholipids, i.e., membrane substrates, may be used singly, or in a combination of two or more. For example, a combination of phosphatidylcholine with phosphatidylglycerol, or a combination of a saturated phospholipid with charged phosphatidic acid, phosphatidylglycerol, dicetyl phosphate, etc., may be used.

In order to prevent drugs enclosed in liposomes from easily leaking from the liposomes during storage or in the body (e.g., blood), it is preferable to use a main membrane material having a phase transition point higher than the in vivo temperature (35 to 37°C). In the production of such liposomes, the liposomes may be exposed to a temperature higher than the in vivo temperature. That is, the liposomes may be produced under temperature conditions of about 50°C to 70°C, for example about 60°C, and the thermal effect on the liposome formation becomes high; therefore, it is particularly preferable to use a main membrane material having a phase transition point higher than these temperatures. More specifically, the phase transition point of the main membrane material is preferably 50°C or higher.

Other membrane components that can maintain the above membrane structure and be contained in liposomes may suitably be used in combination with the above phospholipids (membrane substrates) as long as the object of the present invention is not impaired. Examples of other membrane components include lipids that contain no phosphate group in the molecule, e.g., glyceroglycolipids, sphingoglycolipids, sterols such as cholesterol and cholestanols, and their hydrogenated derivatives, fatty acids, etc.

It is also preferable to use a mixed lipid of a phospholipid and other lipid as a liposome membrane component. For example, a mixed lipid of a phospholipid, cholesterol, and fatty acid is preferably used. The mixing ratio can suitably be determined as long as the form of the liposome capsule is not destroyed. By mixing these components with phospholipids, the phase transition temperature of the phospholipids can be reduced, thus enabling the formation of liposomes at 60°C or less.

To produce liposomes from lipids that contain these phospholipids, membrane constituents including phospholipids are, for example, although not limited thereto, mixed in an organic solvent (e.g., chloroform) in a flask, and the organic solvent is removed, followed by vacuum drying, thereby forming a thin film on the inner wall of the flask. Subsequently, an internal aqueous phase solution is added in the flask, followed by vigorous stirring, thereby obtaining a suspension of liposomes.

With respect to the pH of the internal aqueous phase of the liposome, the internal aqueous phase solution to be added can be adjusted to a desired pH using a pH adjuster etc., as needed. Subsequently, the obtained liposome dispersion is centrifuged, and the supernatant is purified by decantation, thereby obtaining a suspension of liposomes.

In addition to this method, the liposome can also be obtained by mixing the above-described constituents, and then discharging the resultant mixture at a high pressure by means of a high-pressure discharge emulsifier. This method is described in detail in "Liposome in Life Science" (Terada, Yoshimura, et al.; Springer-Verlag Tokyo (1992)). Some technologies are available for sizing of the liposomes to the desired size (edited by G. Gregoriadis, "Liposome Technology Liposome Preparation and Related Techniques" 2nd edition, Vol. I-III, CRC Press). These disclosures are incorporated as part of the specification.

Although the size of the liposome is not limited, the diameter of the average particle outside diameter in a spherical form or a form similar thereto may be 100 to 300 nm, and is preferably about 200 nm. The diameter can be measured by the dynamic light scattering method, for example.

The liposome may contain a drug in the internal aqueous phase. A drug can be supported in the liposome by employing a method according to the drug to be supported. For example, there are the following methods: a passive loading method, in which a drug is supported in a liposome by hydrating a lipid bilayer membrane, which constitutes a liposome, in an aqueous solution containing the drug; and a remote loading method, in which a drug is supported in a liposome by transporting the drug through a liposome membrane according to the ion gradient formed in the inside and outside of the liposome membrane.

The liposome may further contain pharmaceutically acceptable stabilizers, antioxidants, and other additives, depending on the route of administration. Examples of stabilizers include, but are not limited to, substances that reduce the membrane fluidity; for example, saccharides such as glycerol and sucrose. Sterols such as cholesterol, which are referred to above as other lipids of the membrane constituents, act as such stabilizers. Examples of antioxidants include, but are not limited to, ascorbic acid, uric acid or α, β, γ, and δ tocopherol homologs; for example, vitamin E. Examples of additives include water, physiological saline, pharmaceutically acceptable organic solvent, collagen, polyvinyl alcohol, polyvinyl pyrrolidone, carboxyvinyl polymer, sodium carboxymethylcellulose, sodium polyacrylate, sodium alginate, water-soluble dextran, sodium carboxymethyl starch, pectin, methylcellulose, ethylcellulose, xanthan gum, gum arabic, casein, gelatin, agar, diglycerol, propylene glycol, polyethylene glycol, vaseline, paraffin, stearyl alcohol, stearic acid, human serum albumin (HSA), mannitol, sorbitol, lactose, PBS, bioerodible polymer, serum-free medium, surfactant that is acceptable as a pharmaceutical additive, buffer with a physiological pH that is acceptable in vivo, and the like. Although not limited thereto, each of these components may be suitably selected, or may be used in combination.

In the present invention, various drugs can be supported in the liposomes. Examples of drugs include therapeutic agents, such as nucleic acids, polynucleotides, genes, and analogs thereof, anticancer drugs, antibiotics, enzyme agents, antioxidants, lipid uptake inhibitors, hormone drugs, antiinflammatory agents, steroids, vasodilators, angiotensin-converting enzyme inhibitors, angiotensin receptor antagonists, smooth muscle cell growth/migration inhibitors, platelet aggregation inhibitors, anticoagulants, chemical mediator isolation inhibitors, vascular endothelial cell growth promoters or vascular endothelial cell growth inhibitors, aldose reductase inhibitors, mesangial cell proliferation inhibitors, lipoxygenase inhibitors, immunosuppressants, immunomodulators, antiviral agents, Maillard reaction inhibitors, amyloidosis inhibitors, nitric oxide synthesis inhibitors, AGEs (Advanced Glycation End products) inhibitors, radical scavengers, proteins, peptides, glycosaminoglycan, and derivatives thereof, oligosaccharides, polysaccharides, and derivatives thereof; X-ray contrast agents; and in vivo diagnostic products, such as ultrasound diagnostic products, radioisotope-labeled nuclear medicine diagnostic agents, and diagnostic products for nuclear magnetic resonance diagnosis.

The antibody-binding liposome of the invention can be prepared by mixing the liposome suspension prepared in the above manner, with the linker peptide mentioned above. That is, both are mixed, so that the linker peptide binds to the liposome, thereby obtaining the antibody-binding liposome of the invention. Although the mixing conditions are not limited, the mixed state is preferably maintained at room temperature for about 30 minutes to several hours, and preferably about 1 hour. More preferably, the mixed state is maintained at about 22°C for one hour to several hours.

Although the mixing proportion of linker peptide to liposome is not limited, the concentration of linker peptide in a liposome suspension containing the liposome at a ratio of 1.5 wt.% in terms of phospholipid concentration is, for example, 0.01 to 0.05 wt.%, and preferably 0.025 to 0.05 wt.%.

Thus, the linker peptide binds to the liposome at least with the antibody-binding peptide region exposed to the outside of the liposome, and with the liposome-binding peptide region stuck in (or penetrating through) the lipid bilayer membrane of the liposome. The liposome having antibody-binding ability (antibody-binding liposome) of the invention can be obtained in this manner.

### (2) Antibody-Modified Liposome and Preparation Method Thereof

The "antibody-modified liposome" intended by the present invention is a liposome in which antibodies bind to the above-described antibody-binding liposome.

The "antibody" used herein may be antiserum, and is preferably IgG.

The antibody-modified liposome can be prepared by mixing such antibodies with the above-described antibody-binding liposome. More specifically, both are mixed, so that the antibodies are coupled to the antibody-binding liposome via the linker peptide, thereby obtaining the antibody-modified liposome of the invention. Although mixing conditions are not limited, it is preferable to maintain the mixed state at room temperature for about, for example, 30 minutes to several hours, preferably about one hour. It is more preferable to maintain the mixed state at about 22°C for one hour to several hours. The mixing is preferably carried out at a salt concentration of 150 mM or less.

Although the mixing proportion of antibody to antibody-binding liposome is not limited, the concentration of antiserum in a liposome suspension containing the antibody-binding liposome at a ratio of 1.5 wt.% in terms of phospholipid concentration is, for example, 0.01 to 0.1 wt.%, and preferably 0.05 to 0.1 wt.%.

The antibody-modified liposome of the invention labeled with antibodies, particularly IgG, can be prepared in this manner.

The antibody-modified liposome can be prepared as an active targeting-type liposome, which is useful as a drug delivery system preparation (DDS preparation) by using antibodies having a targeting function for target tissues or cells. Thus, the antibody-modified liposome can be provided as a DDS preparation. The use of the active targeting-type liposome allows specific drug delivery to cancer cells and specific delivery of desired antigens to target cells. Since the antibody-modified liposome of the invention can be readily prepared using any antibodies, it can be effectively used as a screening tool during the construction of the above-mentioned DDS preparation (active targeting-type liposome) etc.

### EXAMPLES

The present invention is described in more detail below with reference to examples; however, the invention is not limited thereto.

### Experimental Example 1: Properties of Migrating and Binding to Liposome Membrane

Liposomes were mixed with a peptide (3L-DR peptide) represented by SEQ ID NO: 2, which corresponds to the liposome-binding peptide of the present invention, followed by a digestion experiment using protease. The 3L-DR peptide has a Myc-tag region in a 1 to 10 amino acid region, and a T7-tag region in a 59 to 69 amino acid region.

The liposomes used here were prepared by mixing phosphatidylcholine (PC) with phosphatidylglycerol (PG) at a ratio of 1:3 (molar ratio), and preparing the liposomes in a 10 mM HEPES buffer (pH: 8.5) containing 100 mM of sodium sulfate in accordance with a standard method. The particle size of the liposomes was about 200 nm, which was adjusted by passing the liposomes through a polycarbonate filter with a pore size of 0.4 µm for sizing.

More specifically, the peptide (3L-DR peptide) of SEQ ID NO: 2 was added to a liposome suspension, followed by mixing at room temperature for one hour. Subsequently, trypsin was added thereto so that the concentration was 0.5 mg/ml, and the mixture was kept warm at 37°C for 30 minutes. Meanwhile, as a comparative test, Triton X-100 (surfactant) was added to the above-prepared mixture of liposomes and 3L-DR peptide, in addition to 0.5 mg/ml of trypsin, so that the final concentration was 1%, and the resultant mixture was similarly kept warm at 37°C for 30 minute.

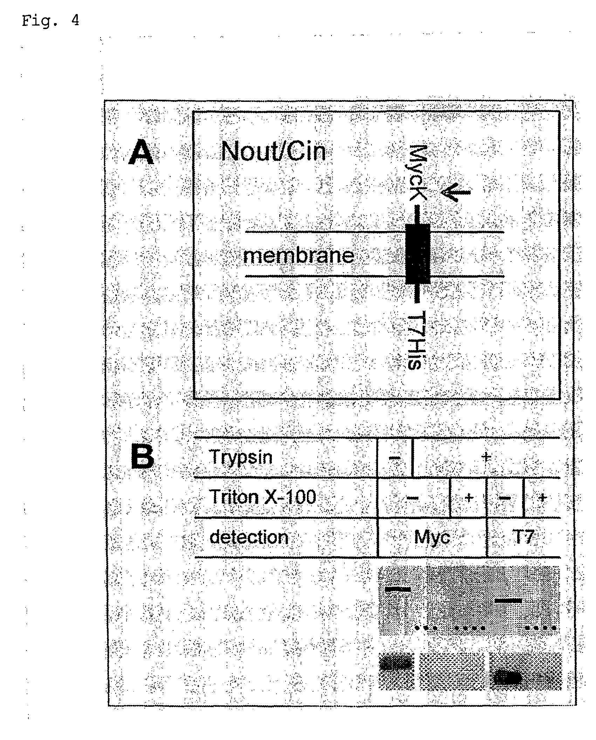

The reaction mixture obtained above was applied to electrophoresis (using 10 to 15% of acrylamide gel; mobile phase: 25 mM Tris, 250 mM glycine, 0.1% SDS, pH 8.3) together with the solution before trypsin treatment (Trypsin(-)). The results are shown in Fig. 4B. Additionally, a sample prepared by adding an equivalent amount of 2 x loading buffer (100 mM Tris-Cl, pH 6.8, 200 mM dithiothreitol, 4% SDS, 0.2% bromophenol blue, 20% glycerol) to the specimen was also applied to electrophoresis.

As is clear from the results, the 3L-DR peptide having a Myc-tag region was detected in the solution before trypsin treatment (Trypsin(-), Triton X-100(-)); whereas in the solution after trypsin treatment (Trypsin(+), Triton X-100(-)), no Myc-tag of the 3L-DR peptide was detected, and a somewhat short peptide having a T7-tag region was observed. This shows that the N-terminal region including the Myc-tag region of the 3L-DR peptide was digested by trypsin treatment. This suggests that the 3L-DR peptide is not simply absorbed onto the surface of the lipid bilayer membrane of the liposome, but is stuck in and bound to the lipid bilayer membrane of the liposome with the Myc-tag region exposed on the liposome surface. In the comparative test, in which trypsin treatment was carried out in the presence of a surfactant (Triton X-100) (Trypsin(+), Triton X-100(+)), neither Myc-tag nor T7-tag of the 3L-DR peptide was detected. The reason for this is considered to be that since the liposomes were destroyed in the presence of the surfactant (Triton X-100), and the peptide therefore became exposed, the 3L-DR peptide was digested by trypsin treatment.

Accordingly, as shown in Fig. 4A, the 3L-DR peptide (SEQ ID NO: 2) is presumably stuck in and bound to the lipid bilayer membrane of the liposome with the N-side region including the Myc-tag region (expressed by "MycK" in the figure) exposed on the liposome surface.

### Experimental Example 2: Binding Property to Liposome

### (1) Preparation of Subject Peptide

Subject peptides used were as follows:
(a) a peptide in which a His-tag (ASHHHHHH: SEQ ID NO: 4) is attached to the C-terminus of the amino acid sequence of SEQ ID NO: 3, derived from one antibody-binding unit (domain B) of Protein A (hereafter referred to as "SPA peptide"; SEQ ID NO: 5), and
(b) a peptide in which a 2 to 75 region of the 3L-DR peptide (SEQ ID NO: 2) is attached to the C-terminus of the SPA peptide from which the His-tag region had been removed (hereinafter referred to as "SPA-DR peptide"; SEQ ID NO: 6; molecular weight: about 16 kDa).

These peptides were prepared in the following manner. Expression vectors for the peptides were prepared using a pET vector or pCold vector. Each of the resulting vectors was introduced into an *E. coli* strain BL21 (DE3), and the *E. coli* cells were cultured in LB medium. IPTG (isopropyl-β-D-thiogalactopyranoside) was added so that the final concentration was 0.4 mM to induce the expression.

### (2) Preparation of Liposome

Liposomes were prepared by mixing phosphatidylcholine (PC) with phosphatidylglycerol (PG) at a ratio of 1:3 (molar ratio), and preparing the liposomes in a 10 mM HEPES buffer (pH: 8.5) containing 100 mM of sodium sulfate in accordance with a standard method. The resulting liposomes were passed through a polycarbonate filter with a pore size of 0.4 µm for sizing so that the particle size was adjusted to about 200 nm.

### (3) Test of Binding to Liposome

Each peptide (10 to 20 µg) was added to the above-prepared liposomes in an amount equivalent to 0.2 µmol, and the mixture was kept warm at 37°C for 1 hour, followed by centrifugation at 105,000 x g at 4°C for 1 hour. Subsequently, the supernatant (S) and the precipitate (P), which contained the liposomes, were separated, and peptides contained in each fraction were detected by western blotting using an anti-His-tag antibody or anti-Myc-tag antibody.

### (4) Results

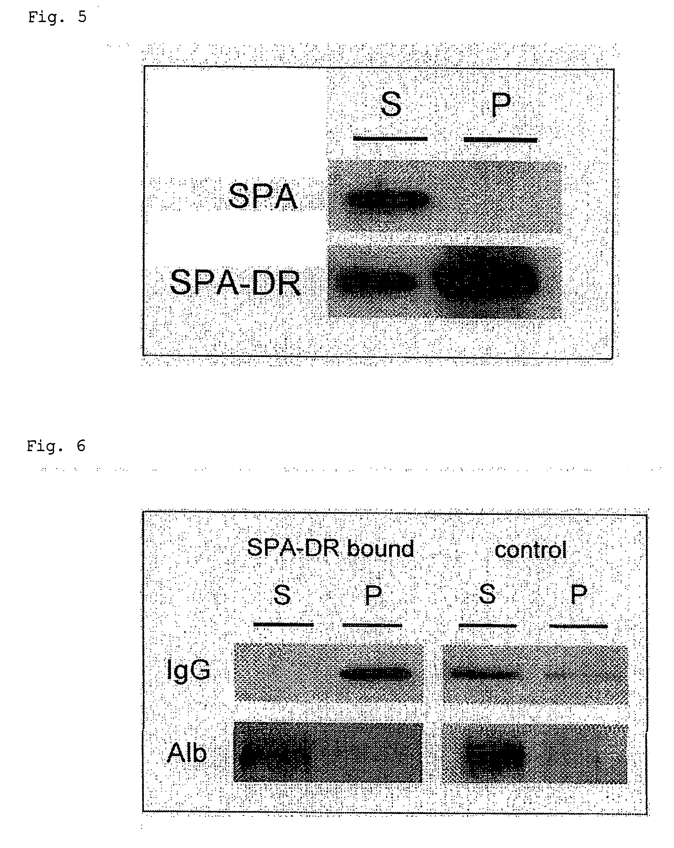

The results of the western blot are shown in Fig. 5. As shown in Fig. 5, in the liposome-containing precipitate (P) fraction, only the SPA-DR peptide was detected, and no SPA peptide was detected. This revealed that the SPA peptide corresponding to one antibody-binding unit of Protein A did not bind to liposomes, and that only the SPA-DR peptide, which was obtained by fusing the 3L-DR peptide with the SPA peptide, bound to liposomes. It was believed from the results that the 3L-DR region of the SPA-DR peptide has the property of binding to liposomes, and that the SPA-DR peptide can thereby bind to liposomes via the 3 L-DR region.

### Experimental Example 3: Binding Property to Antibody

The SPA region of the SPA-DR peptide is an antibody-binding unit of Protein A, and has antibody-binding ability. Therefore, if the liposomes linked to the SPA-DR peptide (SPA-DR-binding liposomes) prepared above are confirmed to have antibody-binding ability, the SPA-DR peptide is considered to be bound to a liposome with the 3L-DR region inserted into the liposome membrane, and the SPA region (antibody-binding unit) exposed on the liposome surface.

In order to confirm this, an experiment of adding antiserum to the SPA-DR-binding liposomes prepared above was carried out, as described below, to examine whether IgG binds to the liposomes.

### (1) Process of Experiment

More specifically, antiserum in an amount equivalent to 10 µg was added to the precipitate (P) fraction (corresponding to P of SPA-DR in Fig. 5) prepared in Experimental Example 2 containing the liposomes, which were confirmed to be linked to the PA-DR peptide, and the mixture was kept warm at 25°C for 1 hour, followed by centrifugation at 105,000 x g at 4°C for 1 hour. Further, as a control test, antiserum in an amount equivalent to 10 µg was similarly added to the precipitate (P) fraction (corresponding to P of SPA in Fig. 5) prepared in Experimental Example 2 containing the liposomes that were obtained by adding SPA, followed by centrifugation. The mixture was kept warm at 25°C for 1 hour, followed by centrifugation at 105,000 x g at 4°C for 1 hour. The antiserum used was rabbit crude serum immunized with partial peptide of human transcription factor PGC1α. Subsequently, IgG and albumin (Alb) contained in each of the supernatant (S) and precipitate (P) fractions separated by centrifugation were detected by western blotting.

### (2) Experimental Results

The results are shown in Fig. 6. In Fig. 6, the left column (SPA-DR bound) shows the results obtained by adding antiserum to the precipitate (P) fraction containing the liposomes, which were confirmed to be linked to the SPA-DR peptide (S: supernatant, P: precipitate), and the right column (control) shows the results obtained by adding antiserum to the liposome fraction obtained by centrifugation after the addition of SPA (S: supernatant, P: precipitate).

The results indicate that the antiserum (IgG) added to the liposome-containing precipitate (P) fraction was not detected in the supernatant (S) fraction, as shown in the left column of Fig. 6. This revealed that all IgG was bound to the liposomes (SPA-DR-binding liposomes) in the precipitate (P) fraction. Moreover, from the fact that almost all albumins in the serum were not bound to the liposomes in the precipitate (P) fraction, it was confirmed that the binding of IgG to the SPA-DR-binding liposomes observed above was not specific.

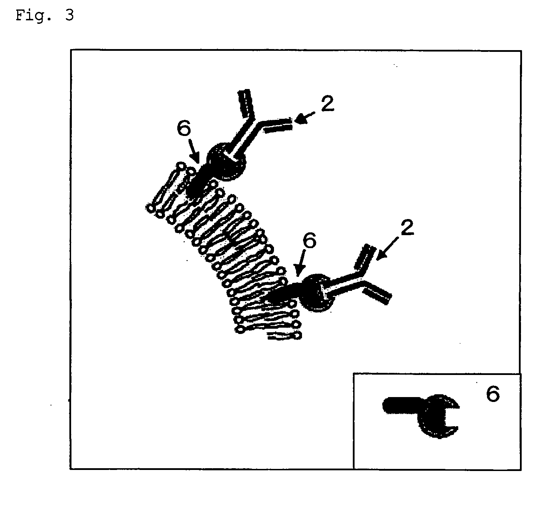

It was confirmed from the results that the SPA-DR peptide was bound to the liposomes with the 3L-DR peptide region (corresponding to the liposome-binding peptide) inserted into the liposome membrane, and with the SPA peptide region (corresponding to the antibody-binding peptide) exposed on the liposome surface, as shown in the diagram of Fig. 3.

### BRIEF DESCRIPTION OF THE DRAWINGS

Fig. 1 is a diagram schematically illustrating an embodiment of well-known modification of the liposome surface with antibodies. Such liposomes are prepared by mixing antibody molecules and phospholipids reactive with antibodies in the presence of a crosslinking reagent. In the figure, signs 1, 2, and 3 denote a "phospholipid reactive with antibodies", "antibody", and "crosslinking reagent", respectively.
Fig. 2 is a diagram schematically illustrating an embodiment of modification of the liposome surface with antibodies using well-known Protein A. In this embodiment, Protein A (sign 4) having antibody-binding ability is crosslinked to a phospholipid (sign 1). The lower right figure is a diagram schematically illustrating Protein A (sign 4) that has five antibody-binding domains (sign 5). Sign 2 denotes an "antibody".
Fig. 3 is a diagram schematically illustrating an embodiment of antibody modification of the liposome surface according to the present invention. The lower right figure is a diagram schematically illustrating the linker peptide (sign 6) of the invention. Sign 2 denotes "antibody".
In Fig. 4, B shows the results of electrophoresis performed in Experimental Example 1, and A is a diagram schematically illustrating a binding form of the liposome-binding peptide to a liposome predicted by the results.
Fig. 5 shows the results of the western blot carried out in Experimental Example 2. In the figure, "SPA" denotes a peptide that is one antibody-binding unit of Protein A, and "SPA-DR" denotes a fusion peptide obtained by binding 3L-DR peptide, which can migrate to the membrane, to the C-terminus of SPA. "S" denotes a supernatant fraction, and "P" denotes a precipitate fraction. The results demonstrate that the SPA peptides do not bind to liposomes, while the fusion peptides obtained by binding 3L-DR peptides to the SPA peptides bind to liposomes.
Fig. 6 shows the results of the western blot carried out in Experimental Example 3. The left column (SPA-DR bound) shows the results obtained by adding antiserum to the precipitate (P) fraction containing liposomes (S: supernatant, P: precipitate), and the right column (control) shows the results obtained by adding antiserum to the supernatant (S) fraction free of liposomes (S: supernatant, P: precipitate).

### SEQUENCE LISTING FREE TEXT

SEQ ID NO: 1 shows the amino acid sequence of the linker peptide.
SEQ ID NO: 2 shows the amino acid sequence of the liposome-binding peptide.
SEQ ID NO: 3 shows the amino acid sequence of the antibody-binding peptide.
SEQ ID NO: 4 shows the amino acid sequence of the His-tag region.
SEQ ID NO: 5 shows the amino acid sequence of the SPA peptide used as a subject peptide in Experimental Example 2 (a peptide derived from one antibody-binding unit (domain B) of Protein A, and obtained by adding the His-tag (SEQ ID NO: 4) to the C-terminus of the amino acid sequence of SEQ ID NO: 3).
SEQ ID NO: 6 show the amino acid sequence of the SPA-DR peptide used as a subject peptide in Experimental Example 2 (a peptide obtained by adding a 2 to 73 region of 3L-DR peptide (SEQ ID NO: 2) to the C-terminus of the SPA peptide from which the His-tag region was removed).

## Claims

1. A liposome-binding peptide consisting of an amino acid sequence (a) or (b):
(a) an amino acid sequence represented by SEQ ID NO: 2,
or
(b) an amino acid sequence derived from the amino acid sequence (a) by deletion, substitution or addition of one or several amino acids with the proviso that the amino acid sequence contains at least 11 to 58 and 70 to 73 amino acid regions, and encoding a peptide that has a liposome-binding property and a property of migrating to a liposome membrane.

2. An antibody-binding peptide consisting of an amino acid sequence (c) or (d):
(c) an amino acid sequence represented by SEQ ID NO: 3,
or
(d) an amino acid sequence derived from the amino acid sequence (c) by deletion, substitution or addition of one or several amino acids, and encoding a peptide that has antibody-binding ability.

3. A linker peptide consisting of an amino acid sequence (e) or (f):
(e) an amino acid sequence represented by SEQ ID NO: 1,
or
(f) an amino acid sequence derived from the amino acid sequence (e) by deletion, substitution or addition of one or several amino acids, and encoding a peptide that has a liposome-binding property and an antibody-binding property.

4. The linker peptide according to claim 3, in which the liposome-binding peptide of claim 1 binds to the C-terminus of the antibody-binding peptide of claim 2.

5. An antibody-binding liposome modified with the linker peptide of claim 3 or 4.

6. A method of preparing the antibody-binding liposome according to claim 5, comprising the step of mixing the linker peptide of claim 3 or 4 with a liposome, thereby binding said linker peptide to the liposome.

7. An antibody-modified liposome in which an antibody binds to the antibody-binding liposome of claim 5 via the linker peptide of claim 3 or 4.

8. The antibody-modified liposome according to claim 7, wherein the antibody is IgG.

9. A method of preparing the antibody-modified liposome according to claim 7 or 8, comprising the step of mixing the antibody-binding liposome of claim 5 with an antibody, thereby binding the antibody to said liposome via the linker peptide of claim 3 or 4.

## Amended claims

### Amended claims under Art. 19.1 PCT

**1.** A liposome-binding peptide consisting of an amino acid sequence (a) or (b):
(a) an amino acid sequence represented by SEQ ID NO: 2, or
(b) an amino acid sequence derived from the amino acid sequence (a) by deletion, substitution or addition of one or several amino acids with the proviso that the amino acid sequence contains at least 11 to 58 and 70 to 73 amino acid regions, and encoding a peptide that has a liposome-binding property and a property of migrating to a liposome membrane.

**3.** A linker peptide consisting of an amino acid sequence (e) or (f):
(e) an amino acid sequence represented by SEQ ID NO: 1, or
(f) an amino acid sequence derived from the amino acid sequence (e) by deletion, substitution or addition of one or several amino acids, and encoding a peptide that has a liposome-binding property and an antibody-binding property.

**4.** (Amended) The linker peptide according to claim 3, in which the liposome-binding peptide of claim 1 binds to the C-terminus of the antibody-binding peptide consisting of an amino acid sequence (c) or (d):
(c) an amino acid sequence represented by SEQ ID NO: 3, or
(d) an amino acid sequence derived from the amino acid sequence (c) by deletion, substitution or addition of one or several amino acids, and encoding a peptide that has antibody-binding ability.

**5.** An antibody-binding liposome modified with the linker peptide of claim 3 or 4.

**6.** A method of preparing the antibody-binding liposome according to claim 5, comprising the step of mixing the linker peptide of claim 3 or 4 with a liposome, thereby binding said linker peptide to the liposome.

**7.** An antibody-modified liposome in which an antibody binds to the antibody-binding liposome of claim 5 via the linker peptide of claim 3 or 4.

**8.** The antibody-modified liposome according to claim 7, wherein the antibody is IgG.

**9.** A method of preparing the antibody-modified liposome according to claim 7 or 8, comprising the step of mixing the antibody-binding liposome of claim 5 with an antibody, thereby binding the antibody to said liposome via the linker peptide of claim 3 or 4.
